# EUROPEAN PATENT APPLICATION

(11) **EP 0 755 697 A2**
(43) Date of publication of application: **29.01.1997**
(21) Application number: 96305359.0
(22) Date of filing: 22.07.1996
(51) Int. Cl.: A61N 5/06

(54) **Cancer therapeutic instruments**

(30) Priority: 28.07.1995 JP 193765/95
(71) Applicant: Hashimoto, Yasuo, Yokohama-shi, Kanagawa-ken 247 (JP); Yamaguchi, Noboru, Yokohama-shi, Kanagawa-ken 235 (JP)
(72) Inventor: Hashimoto, Yasuo, Yokohama-shi, Kanagawa-ken 247 (JP); Yamaguchi, Noboru, Yokohama-shi, Kanagawa-ken 235 (JP)
(74) Representative: Overbury, Richard Douglas

(57) **Abstract**

There is provided a cancer therapeutic instrument, according to a light therapy using laser beams and the like, capable of performing a medical treatment while cooling the affected part. A catheter type of insertion member 80 has a tube inserted into the affected part, and an optical fiber the tip portion of which is disposed inside the tube at the position associated with the affected part. The tube penetrates the affected part and extended to the left. The tube is provided with a branch for supplying a cooling medium of liquid or gas to the tube. The cooling medium fed from the branch flows in a direction of arrows A-A'(Fig. 2), so that the tube and the affected part being in contact with the tube are cooled. After cooling the tube and the affected part, the cooling medium is collected through the tip portion (not illustrated) of the tube extended toward the left direction.

## Description

### BACKGROUND OF THE INVENTION

### Field of the Invention

The present invention relates to a cancer therapeutic instrument for use in treatment for cancers and neoplasm, and more particularly a cancer therapeutic instrument for use in treatment by light therapy for the cancers and neoplasm originated, for example, in an area which involves a great deal of difficulty in performing resection operation, the organs and the like, for instance, pancreatic carcinoma, lung cancer, brain tumor, cancers originated in the brain stem area and the vertebral column, and cancer originated in the neighborhood of the aorta.

### Description of the Related Art

Currently, a cancer is one of the most terrible diseases which are left to mankind, and elucidation of its fundamental cause and the therapy are in a developing state.

As typical therapy, which is considered at present to be effective, surgical resection way, radiation therapy and carcinostatic agent therapy are well known and most frequently employed.

Among those therapeutic ways, the surgical resection way is deemed to be most effective and is most frequently employed. On the other hand, for malignant tumor originated in the deep area of the body for which the surgical resection way cannot be employed, there is frequent such a case that radiation therapy and carcinostatic agent therapy are used in their combination. However, it often happens that both radiation therapy and carcinostatic agent therapy involve strong adverse reaction, and the radiation therapy and carcinostatic agent therapy are remarkably lower in complete cure rate as compared with the surgical resection way and involve danger of relapses.

Recently, in view of the foregoing, there is hastened vigorously a study of cancer therapy according to physical therapy which involves no adverse reaction. As typical therapy, there are raised hyperthermia and light therapy using a photosensitizer.

The hyperthermia is involved in a way such that a cancer cell is extinguished through heating and/or cooling thereof. It has been reported that keeping a cancer cell at a temperature, for example, higher by + 5 °C than a body temperature, which would cause merely relatively less damage to peripheral normal cells, throughout a definite time, alone may bring a great treatment effect.

On the other hand, the light therapy is based on a such a mechanism that irradiation with a specified wavelength of activation light on a cell absorbing a photosensitizer leads to an absorption of optical energy by the photosensitizer, so that activating oxygen, which will destroy the cell, emanates. This treatment utilizes the fact that an excretion ratio of the photosensitizer after injection thereof is extremely lower in a cancer cell as compared with a normal cell, specifically, less than 1/3 (about 1/5).

The present invention is involved in the light therapy, and thus the light therapy will be described in detail hereinafter.

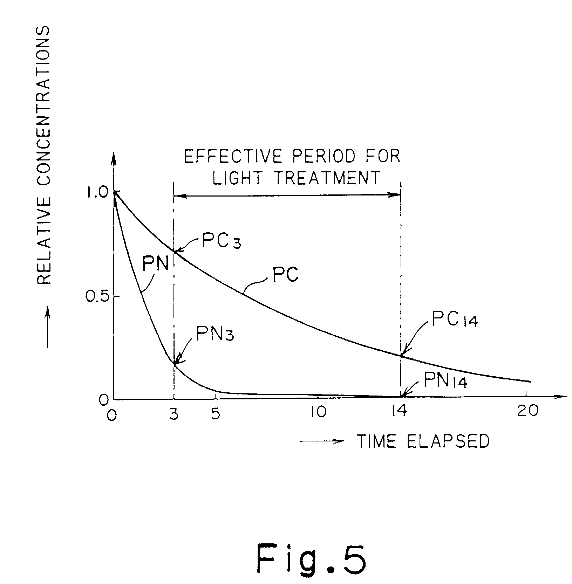

Fig. 5 is a graph showing a relation between time elapsed wherein a time point when an ATX-S10, which is one of photosensitizers, is injected into the body, is selected as a starting point and the relative concentrations of the ATX-S10 remaining in cells. The graph PN denotes the residual concentrations of the ATX-S10 within the normal cells; and the graph PC the residual concentrations of the ATX-S10 within the cancer cells.

When a certain standby time (about 3 hours) lapses after a plenty of photosensitizer is injected into the affected part and its peripheral cells through a direct injection of the photosensitizer into the affected part, an intravenous injection and the like, the normal cells have discharged almost all photosensitizer, whereas there appears such a state (light treatment feasible state) that almost all photosensitizer stays in the cancer cells. In such a light treatment feasible state, irradiation with a specified wavelength of activation light leads to an emanation of activating oxygen, which will destroy the cells, within the cancer cells in which almost all photosensitizer stays, while no activating oxygen almost emanates within the normal cells which have discharged almost all photosensitizer, whereby the cancer cells are selectively extinguished.

Duration (the term of validity for light treatment) of the light treatment feasible state after an injection of the photosensitizer starts after 3 hours since injection of the photosensitizer and terminates after 14 hours with which the cancer cells have discharged almost all photosensitizer. Incidentally, Fig. 5 shows an ATX-S10 by way of example. The standby time after injection of the photosensitizer and the term of validity for light treatment will vary about one order of magnitude in accordance with a sort of the photosensitizer.

The gist of this treatment resides in the point that activation light is continuously projected onto the affected part as uniformly as possible during the term of validity for light treatment, and an amount of irradiation light is controlled in such a manner that cell-inside-concentrations of activating oxygen which causes cells to be extinguished is restricted to a very low value such that death rate of the normal cells is in the permitted limit within the normal cells, whereas the cell-inside-concentrations of activating oxygen assumes a value such that death rate of the cancer cells is near upon 100% within the cancer cells.

To project the activation light onto the affected part, hitherto, an optical fiber is directly inserted into the affected part and activation light is transmitted through the optical fiber. However, according to this scheme:
(a) Since the optical fiber cannot be moved during the treatment, irradiation area or the therapeutic area is extremely limited;
(b) To project light from the tip of the optical fiber in all directions, it is necessary to cut the tip of the optical fiber, for example, in a cone shape. Processing for such an optical fiber is difficult and then the optical fiber is very expensive;
(c) To perform the treatment repeatedly, it is necessary to do once more insertion of the optical fiber whenever the treatment is performed. It is a great burden to both a patient and a doctor;
(d) Since the therapeutic area is narrow, it is difficult to cure completely large cancer and tumor; and
(e) There are needs of sterilization of the optical fiber before use and re-sterilization of the optical fiber after each use or solid waste disposal. Usually, it is needed to provide an optical fiber having a length not less than 1m, and the optical fiber is of a compound material. Consequently, the sterilization and the solid waste disposal are troublesome and thus the optical fiber is poor in operational efficiency.

In view of the foregoing, the applicants proposed in Japanese Patent Application No. 051950/1994, a cancer therapeutic instrument capable of obtaining a great effect in light treatment through irradiation with activation light on the affected part over a wide range. The cancer therapeutic instrument is excellent in operational efficiency.

Hereinafter, there will be explained the cancer therapeutic instrument proposed in Japanese Patent Application No. 051950/1994.

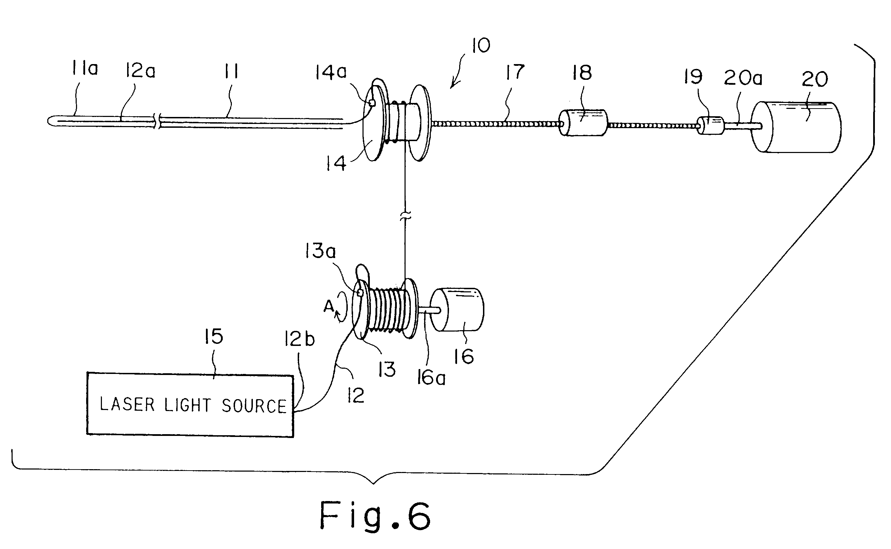

Fig. 6 is a typical illustration of a cancer therapeutic instrument according to an embodiment of the above-proposed invention.

In Fig. 6, the cancer therapeutic instrument 10 is equipped with a throw-away type of tube 11 the tip portion 11a of which is inserted into the affected part involved in the cancer or the tumor within the body of the patient. The tube 11 is made of, for example, polyacetal and is of a diameter of the order of about 1mm.

The cancer therapeutic instrument 10 is further equipped with an optical fiber 12 which is wound into a first reel 13 and a second reel 14 and is fixed on the first reel 13 and the second reel 14 through fixtures 13a and 14a, respectively. The tip portion 12a of the optical fiber 12 at the end of the second reel 14 is inserted into the tube 11 and reaches the tip portion 11a. An incident end 12b of the optical fiber 12 is connected to a laser light source 15. The laser light source 15 emits a beam of laser light having a wavelength utilized as the activation light. The emitted beam of laser light is introduced to the optical fiber 12 through the incident end 12b of the optical fiber 12. The laser light source 15 can emit laser light which is variable in a quantity of light. The quantity of light is varied with the elapse of time by a control circuit (not illustrated). Details will be described later. The laser beam introduced to the optical fiber 12 travels through the optical fiber 12 and emanates from the tip inserted into the tube 11.

The first reel 13 is coupled with a shaft 16a of a torque motor 16 so as to be actuated in an arrow direction A shown in the figure. Fixed on the second reel 14 is a rod 17 over the surface of which male screws are formed. The rod 17 is engaged with a fixed bearing 18 on a spiral basis, and is coupled through a coupling member 19 to a shaft 20a of a reversible motor 20. The fixed bearing 18 is fixed on a base or the like (not illustrated). The reversible motor 20 is rotatable both forward and backward. Providing a guide member (not illustrated) prevents the motor main body from being rotated, and permits the motor to overall move forward and backward (left and right in the figure).

When the reversible motor 20 rotates forward, the rod 17 also rotates forward. Thus, since the rod 17 is engaged with the fixed bearing 18 on a spiral basis, the rod 17 moves forward together with the reversible motor 20, so that the second reel 14 is rotated and advanced. At that time, the second reel 14 pulls the optical fiber 12 wound into the first reel 13 so as to be wound into the second reel 14. Whereas the tip portion 12a of the optical fiber 12 is squeezed into the tube 11, while the optical fiber 12 is rotated forward chosing a longitudinal direction of the optical fiber 12 as an axis. On the other hand, when the reversible motor 20 rotates backward, the rod 17 also rotates backward and steps back. As a result, part of the optical fiber 12, which has been wound into the second reel 14, is rewound into the first reel 13, so that the tip portion 12a of the optical fiber 12 rotates reversely and moves in such a direction that it is drawn from the tube 11. The control circuit (not illustrated) for controlling the reversible motor 20 is provided with a timer. The reversible motor 20 switches over repeatedly the forward rotation and the backward rotation whenever the timer counts up, whereby the tip portion 12a of the optical fiber 12 repeats periodical reciprocation and rotational motion within the tube 11.

Incidentally, according to the present embodiment as described above, there is shown an example in which both the reciprocation and the rotational motion are applied to the tip portion 12a of the optical fiber 12. However, it is noted that modifications can be made. For example, if the fixed bearing 18 is removed and the reversible motor 20 is fixed, the tip portion 12a of the optical fiber 12 performs only the rotational motion. Further, if the fixed bearing 18 is removed, and in addition the rod 17 is coupled with the tip portion 12a of the optical fiber 12 through a gear assembly or the like, with which the coupling member 19 is replaced, for converting the rotational motion of the shaft 20a of the reversible motor 20 into the linear motion, it is possible to provide for the tip portion 12a of the optical fiber 12 only the reciprocation with respect to the longitudinal direction of the tube.

To use the cancer therapeutic instrument 10 shown in Fig. 6, photosensitizer is injected into the affected part, the tube 11 is inserted into the affected part, the tip portion 12a of the optical fiber 12 is inserted into the tube 11, and operation is initiated after a predetermined standby time lapses.

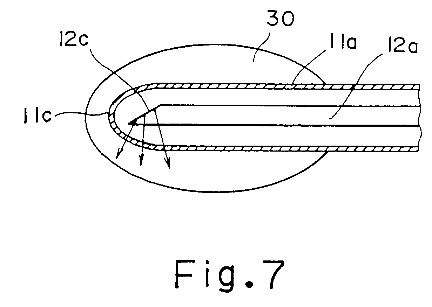

Fig. 7 is a partially enlarged view showing the tip portion 11a of the tube 11 inserted into the affected part 30 and the tip portion 12a of the optical fiber 12 inserted into the tube 11.

As seen from Fig. 7, the tip 11c of the tip portion 11a of the tube 11 is closed so as to prevent a body fluid from invading the inside. The tip of the tip portion 12a of the optical fiber 12, which is inserted up to the tip portion 11a of the tube 11, is provided with the tip plane 12c formed by cutting the tip obliquely as shown in the figure. The laser beam traveled through the inside of the optical fiber 12 is reflected by the tip plane 12c and be emitted through the side plane of the tube 12, so that the affected part 30 is irradiated with the laser beam emitted through the side plane of the tube 12.

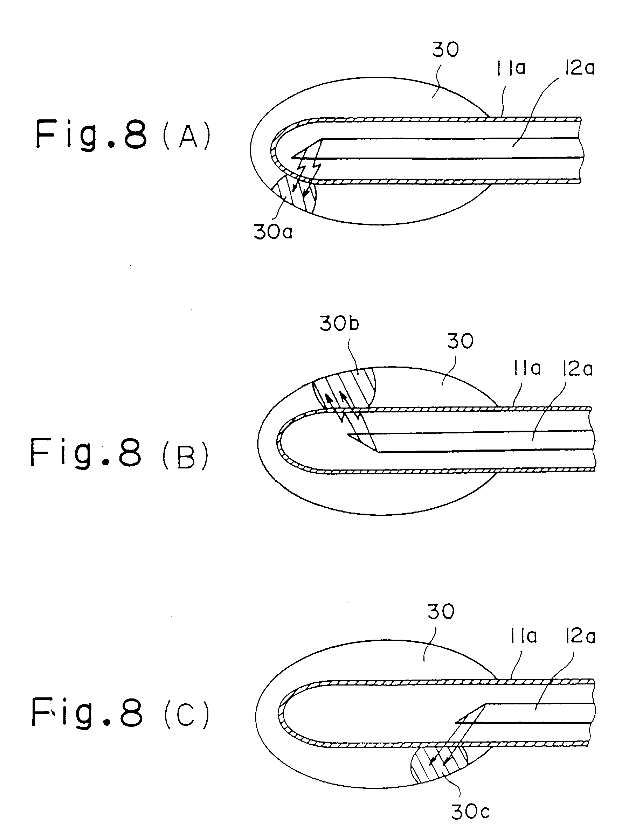

Figs. 8(A)-8(C) are each a view useful for understanding a variation of irradiation area of laser beams according to movement of the optical fiber.

In a state shown in Fig. 8(A), a partial area 30a of the affected part 30 is irradiated with the laser beam. In this state, the tip portion 12a of the optical fiber 12 is rotated and moved in such a direction that it is drawn from the tube 11. At that time, another partial area 30b of the affected part 30 is irradiated with the laser beam as shown in Fig. 8(B). When the tip portion 12a of the optical fiber 12 is rotated and moved to assume the state shown in Fig. 8(C), additional partial area 30c of the affected part 30 is irradiated with the laser beam. In this manner, reciprocation and rotation of the tip portion 12a of the optical fiber 12 inside the tube 11 permits the affected part 30 to be repeatedly irradiated with the laser beams throughout the affected part 30 uniformly.

By the way, if the cancer therapeutic instrument constructed as mentioned above is continuously used for a treatment, the affected part irradiated with the laser beam and the portion of the tube to which the tip of the optical fiber is inserted will begin to be heated. The further continuous irradiation of the laser beam brings about a solidification of blood on the affected part or change of protein in quality, and then carbonization of tissue of the affected part. The carbonized tissue is easy to absorb heat and degrades in thermal conductivity. Thus, a generation of heat is facilitated, thereby forming a vicious circle. A change in quality of the tissue on the affected part begins from the vicinity of the tube which is highest in the density of irradiation of light beam. This involves a degradation in permeability of the laser beam into the affected part, thereby gradually narrowing a treatment area.

Further, the the above-mentioned throw-away type of tube made of, for example, polyacetal or the like, is low in heat resistance property, and thus is easy to melt or change in quality by heat on the tip of the optical fiber. It may happen that the tube springs a leak. When the tube springs a leak, blood may flow into the tube. This causes a solidification of blood and a carbonization, thereby degrading permeability in light. Thus, the effect of treatment is prevented.

In view of the foregoing, it is obliged to use a low output of laser beam through stopping the laser output of the light source down. This involves such a problem that a sufficient treatment effect cannot be expected. It is said that a normal cell can put up with a quantity of light up to the order of 0.2W/cm², 200J. And in order to implement an effective treatment for cancer, there is a need to perform the treatment with increasing a quantity of light per unit area as much as possible. Indeed, however, a quantity of light more than 0.2W/cm² involves heating on a tissue of the periphery of the tube. This causes a change in quality of a tissue and a carbonization, thereby degrading permeability in light on a tissue. Thus, it is obliged to use a low output of the laser beam source.

For these reasons, there is desired a cancer therapeutic instrument capable of obtaining a great effect in treatment using a laser beam of a high output as much as possible.

### SUMMARY OF THE INVENTION

In view of the foregoing, it is therefore an object of the present invention to provide a cancer therapeutic instrument, according to a light therapy, capable of using a high output of laser beam.

To attain the above-mentioned object of the present invention, according to the present invention, there is provided a cancer therapeutic instrument comprising:
a catheter type of insertion member having a tube to be inserted into a subject to be treated, and an optical fiber of which a tip end is adapted to be disposed inside said tube; and
a light source for emitting a predetermined activation light, said light source being arranged to introduce the activation light into said optical fiber,
wherein said tube has a cooling means for cooling a portion in which a tip of said optical fiber inserted into the tube is disposed.

In the instrument as mentioned above, it is preferable that said cooling means is arranged to supply a cooling medium into said tube and collect the supplied cooling medium.

Further, in the instrument as mentioned above, it is preferable that said cooling medium is a liquid having a boiling point between between 25 °C and 80 °C.

Further more, in the instrument as mentioned above, it is preferable that said cooling means comprises a hetero conductor junction constituted of at least two types of conductor, and a power source for supplying a current to said hetero conductor junction, and that said cooling means cools the portion in which the tip of said optical fiber inserted into the tube is disposed, utilizing Peltier effect involved in the current flowing through the hetero conductor junction.

Incidentally, in the cancer therapeutic instrument according to the present invention, it is acceptable that the optical fiber inserted into the tube is to perform a reciprocation and/or a rotational motion.

### BRIEF DESCRIPTION OF THE DRAWINGS

Fig. 1 is a typical illustration of a cancer therapeutic instrument according to the first embodiment of the present invention;
Fig. 2 is a typical illustration of a cancer therapeutic instrument according to the second embodiment of the present invention;
Fig. 3 is a typical illustration of a cancer therapeutic instrument according to the third embodiment of the present invention;
Fig. 4 is a typical illustration of a cancer therapeutic instrument according to the fourth embodiment of the present invention;
Fig. 5 is a graph showing a relation between time elapsed wherein a time point when photosensitizer is injected into the body is selected as a starting point and the relative concentrations of the photosensitizer remaining in cells.
Fig. 6 is a typical illustration of a cancer therapeutic instrument by way of example;
Fig. 7 is a partially enlarged view showing the tip portion of a tube inserted into the affected part and the tip portion of an optical fiber inserted into the tube; and
Figs. 8(A)-8(C) are each a view useful for understanding a variation of irradiation area of laser beams according to movement of the optical fiber;

### DESCRIPTION OF THE PREFERRED EMBODIMENTS

Hereinafter, there will be described embodiments of the present invention.

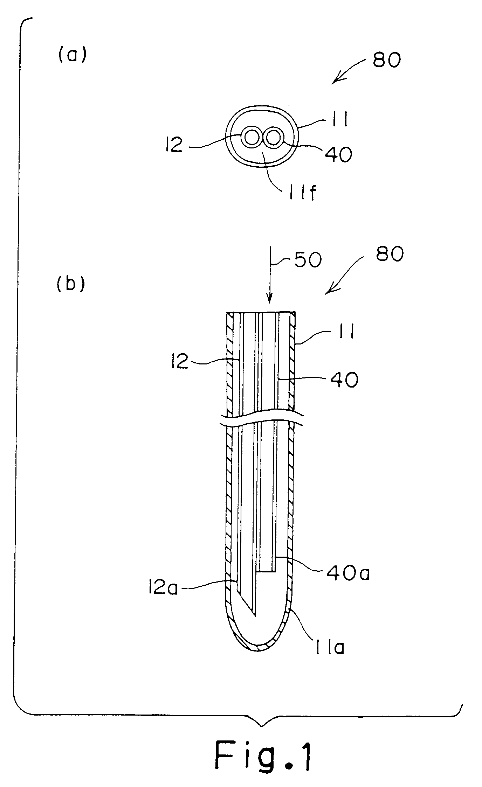

Fig. 1 is a typical illustration of a cancer therapeutic instrument according to the first embodiment of the present invention.

Parts (a) and (b) of Fig. 1 show a transverse section and a longitudinal section of a catheter type of insertion member 80, respectively. The catheter type of insertion member 80 comprises a tube 11 the tip portion 11a of which is closed, an optical fiber 12 the tip portion 12a of which is inserted into the tube 11, and a cooling medium supplying pipe 40 for supplying a cooling medium 50 to the tip portion lla of the tube 11. The cooling medium supplying pipe 40 and the cooling medium 50 supplied thereto constitute a cooling means for cooling the tip portion 11a of the tube 11.

Of the catheter type of insertion member 80, first, the tube 11 is inserted into the affected part 30 of a subject to be treated (cf. Fig. 7), and then the tip portion 12a of the optical fiber 12 is inserted into the tip portion 11a of the tube 11.

The cooling medium 50 is supplied through the cooling medium supplying pipe 40 to the tip portion 11a of the tube 11. The cooling medium 50 serves to take the heat of the affected part 30 (cf. Fig. 7) and the tip portion 11a of the tube 11 by the thermal conduction in the vicinity of the tip portion 11a of the tube 11, so that the affected part 30 and the tip portion 11a of the tube 11 are cooled. After cooling the affected part 30 and the tip portion 11a of the tube 11, the cooling medium 50 is collected from the vicinity of the tip portion 11a of the tube 11 via a vacancy 11f through the rear end (not illustrated) of the tube 11.

With respect to the cooling medium 50, any substances are available for the cooling medium, if it is either liquid or gas excellent in fluidity. From a point of view of cooling efficiency, however, it is preferable that the substance is of high thermal conductivity as much as possible.

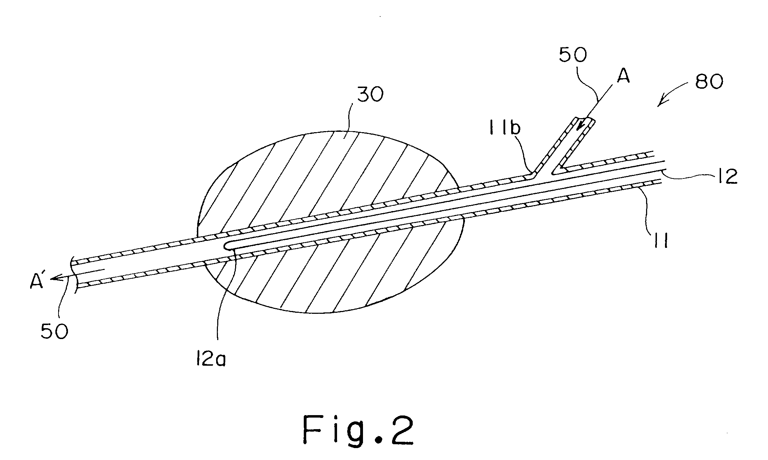

Fig. 2 is a typical illustration of a cancer therapeutic instrument according to the second embodiment of the present invention;

Fig. 2 shows a catheter type of insertion member 80 comprising a tube 11 inserted into the affected part 30, and an optical fiber 12 the tip portion of which is disposed inside the tube 11 at the position associated with the affected part 30. The tube 11 is opened in its both ends, as shown in Fig. 2, and penetrates the affected part 30. This is different from the structure shown in Fig. 1 in which the tip portion 11a of the tube 11 is closed, and as shown in Fig. 7 the closed tip portion 11c is inserted up to the site of the affected part 30.

The tube 11 is provided with a branch 11b for supplying the cooling medium 50 of liquid or gas to the tube. The cooling medium 50 fed from the branch 11b flows in a direction of arrows A-A', so that the tube 11 and the affected part 30 being in contact with the tube 11 are cooled. After cooling the tube 11 and the affected part 30, the cooling medium 50 is collected through the tip portion (not illustrated) of the tube 11 extended toward the left direction.

The use of such a tube 11, in which only one direction is given in a direction of flow of the cooling medium 50 within the tube 11, may provide a smooth flow of the cooling medium 50 thereby expecting a high cooling effect. Further, the arrangement mentioned above may omit the cooling medium supplying pipe 40 as shown in Fig. 1. This feature makes it possible to reduce the tube in thickness. Thus, it is possible to reduce the burden to the patient.

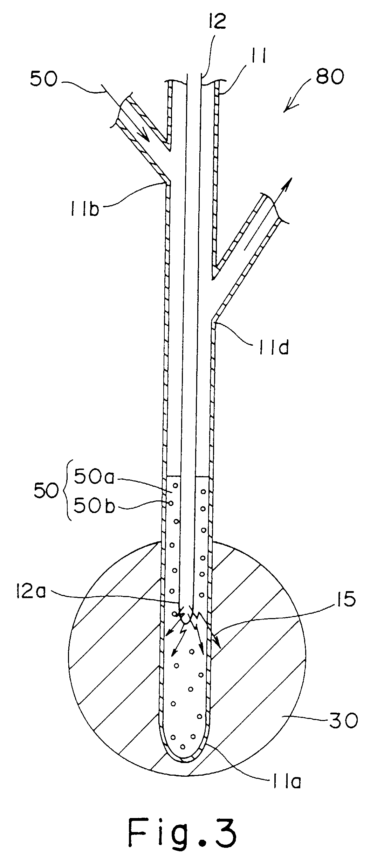

Fig. 3 is a typical illustration of a cancer therapeutic instrument according to the third embodiment of the present invention.

Fig. 3 shows a catheter type of insertion member 80 comprising a tube 11 the tip portion 11a of which is closed, and an optical fiber 12 the tip portion 12a of which is inserted into the tube 11. The tube 11 has a branch 11b for supplying a cooling medium 50a of liquid to the tube 11 and a branch 11d for discharging a cooling medium 50b vaporized out of the tube 11. The optical fiber 12 serves to radiate laser beams 15 to the affected part.

Such an insertion member 80 is kept, as shown in Fig. 3, on a position that the branches 11b and 11d of the tube 11 are located at the upper side, while the tip portion 11a of the tube 11 are located at the lower side. In this state, the tip portion 11a of the tube 11 is inserted into the affected part 30.

When the cooling medium 50a of liquid is supplied through the branch 11b, the cooling medium 50a is temporarily stored in the tube 11. The cooling medium 50a is heated and vaporized by heat emanated from the affected part 30 of the periphery of the tube 11 which is heated by light beams emitted from the tip portion 12a of the optical fiber 12. Thus, the affected part 30 is cooled through the tube 11 by the latent heat of the vaporization which is generated when the cooling medium 50a is vaporized. The cooling medium 50b thus vaporized rises within the tube 11 and is discharged through the branch 11d out of the system.

As the cooling medium 50a, there is used liquid having the boiling point between 25 °C and 80 °C. The reason why the boiling point 25 °C or more is selected is that one which assumes liquid at a normal temperature is easy to be treated. While the reason why the boiling point 80 °C or less is selected is that a plastic material such as polyacetal, which is used in the tube 11, is not changed in quality, usually, if the temperature is 80 °C or less.

As those cooling medium, for example, there may be raised the substances as shown in Table 1.

**Table 1**

| substances | boiling point(°C) | heat of vaporization (cal/g) |
|---|---|---|
| acetone | 56.3 | 125 |
| ethyl alcohol | 78.3 | 200 |
| diethyl ether | 34.5 | 84 |
| carbon tetrachloride | 76.7 | 46.4 |
| ethyl bromide | 38.4 | 58.3 |
| bromide | 58.8 | 43.5 |
| methyl alcohol | 64.7 | 263 |

Incidentally, it is acceptable that the insertion member 80 having a structure in which the tube 11 is extended through the affected portion 30, as shown in Fig. 2, is used to perform a cooling utilizing a latent heat of vaporization of the cooling medium of liquid as shown in Fig. 3.

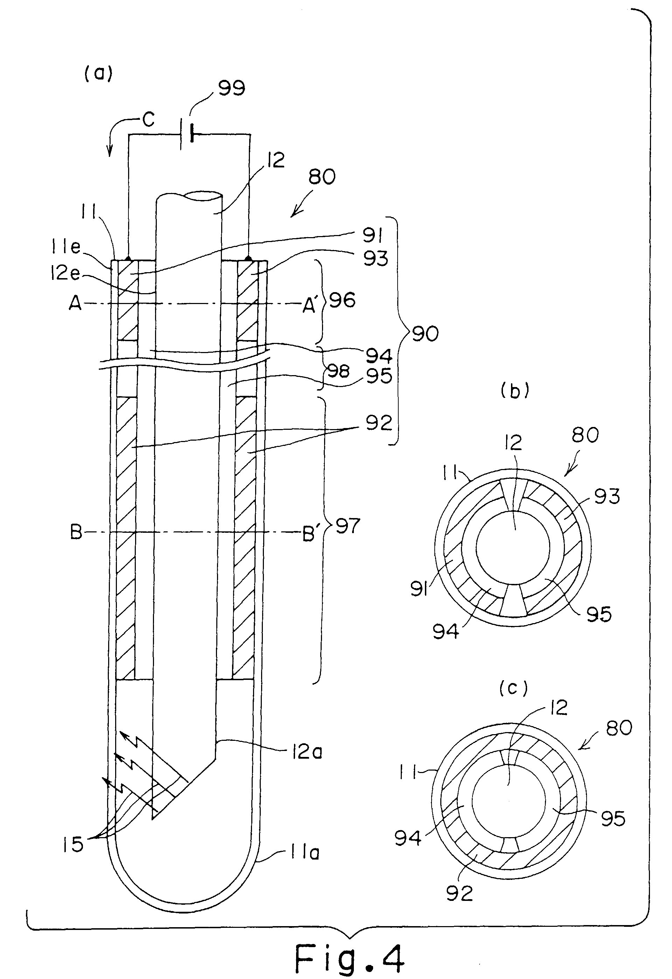

Fig. 4 is a typical illustration of a cancer therapeutic instrument according to the fourth embodiment of the present invention. Part (a) of Fig. 4 shows a longitudinal section of thereof, part (b) of Fig. 4 shows a sectional view taken along the line A-A' of part (a) of Fig.4 and part (c) of Fig. 4 shows a sectional view taken along the line B-B' of part (a) of Fig.4.

As shown in Fig.4, a catheter type of insertion member 80 comprises an optical fiber 12, a conductor tube 90 surrounding the optical fiber 12, a tube 11 surrounding throughout the conductor tube 90, and a power supply 99 for supplying a direct current to a hetero conductor junctions 96 and 97.

The conductor tube 90 comprises the hetero conductor junction 97 used for a heat-absorption provided in the vicinity of the tip portion 11a of the tube 11, the hetero conductor junction 96 used for a heat-generation provided at the rear portion 11e of the tube 11, and an intermediate portion 98 elongated between the hetero conductor junction 96 and the hetero conductor junction 97.

Hitherto, it is well known as Peltier effect that when a direct current flows through a hetero conductor junction constituted of at least two types of conductor, generation or absorption of heat other than Joule heat occurs on the hetero conductor junction. A cooling means according to the present embodiment applies such Peltier effect to the cooling of the tip portion of the tube of the cancer therapeutic instrument.

Specifically, the hetero conductor junction 96 used for a heat-generation is arranged, as shown in part (b) of Fig. 4, in such a manner that the optical fiber 12 is surrounded by an N-type of semiconductor 94 and a P-type of semiconductor 95 each having the configuration which is formed when a cylinder is divided into two pieces, the N-type of semiconductor 94 is surrounded by a first conductor 91 electrically connected to the N-type of semiconductor 94, and the P-type of semiconductor 95 is surrounded by a third conductor 93 electrically connected to the P-type of semiconductor 95.

On the other hand, the hetero conductor junction 97 used for a heat-absorption is arranged, as shown in part (c) of Fig. 4, in such a manner that the optical fiber 12 is surrounded by an N-type of semiconductor 94 and a P-type of semiconductor 95 each having the configuration which is formed when a cylinder is divided into two pieces, and the N-type of semiconductor 94 and the P-type of semiconductor 95 are surrounded by a second conductor 92 electrically connected to the N-type of semiconductor 94 and the P-type of semiconductor 95.

The intermediate portion 98 of the conductor tube 90 comprises an N-type of semiconductor 94 and a P-type of semiconductor 95 each having the configuration which is formed when a cylinder is divided into two pieces, the N-type of semiconductor 94 and the P-type of semiconductor 95 surrounding the optical fiber 12.

The catheter type of insertion member 80 thus formed is inserted into the body of a patient in such a manner that the hetero conductor junction 97 used for a heat-absorption is located in the affected part and the hetero conductor junction 96 used for a heat-generation is located outside the body of the patient.

In this condition, the affected part is irradiated with the laser beams 15 from the tip portion 12a of the optical fiber 12, and in addition a direct current is supplied from the power source 99 to the hetero conductor junctions 96 and 97 in a direction of arrow C. As a result, the current flows from a positive pole of the source through the conductor 91, the N-type of semiconductor 94, the conductor 92, the P-type of semiconductor 95, and the conductor 93 to the negative pole of the source in the names order. Thus, the hetero conductor junction 97 used for a heat-absorption absorbs heat of the periphery of the hetero conductor junction 97, while the hetero conductor junction 96 used for a heat-generation serves to discharge outside the body the heat absorbed by the hetero conductor junction 97. In this manner, the affected part and the tip portion of the tube 11 cool.

As described above, according to the cancer therapeutic instrument of the present invention, a cooling means, which is provided on a tube constituting a catheter type of insertion member, cools the tip portion of an optical fiber inserted into the tube, and absorbs heat of the affected part and the tip portion of the tube generated by the laser beams irradiated from the tip portion of the optical fiber, thereby preventing changes of the affected part and the tube in quality and a carbonization thereof due to heat. Thus, according to the present invention, it is possible to obtain a cancer therapeutic instrument capable of performing effective medical treatment using a high output of laser beam necessary for the treatment.

Further, in the event that the above-mentioned cooling means is arranged in such a manner that a cooling medium is supplied to the tip portion of the optical fiber inserted into the tube and the supplied cooling medium is collected, it is permitted to continuously supply the previously cooled cooling medium. This feature makes it possible to perform effective cooling. Thus, according to the present invention, it is possible to obtain a cancer therapeutic instrument capable of performing effective medical treatment using a high output of laser beam necessary for the treatment.

Furthermore, in the event that the above-mentioned cooling medium is given with liquid having the boiling point between 25 °C and 80 °C, it is possible to utilize the latent heat of a vaporization of the liquid. This feature makes it possible to perform more effective cooling as compared with the case in which only the thermal conduction is utilized. Thus, according to the present invention, it is possible to obtain a cancer therapeutic instrument capable of performing effective medical treatment using a high output of laser beam necessary for the treatment.

Still further, in the event that the above-mentioned cooling means is arranged utilizing Peltier effect involved in the current flowing through the hetero conductor junctions, there is no need to supply the cooling medium. Thus, according to the present invention, it is possible to obtain a cancer therapeutic instrument which is simple in arrangements and easy in operation.

While the present invention has been described with reference to the particular illustrative embodiments, it is not to be restricted by those embodiments but only by the appended claims. It is to be appreciated that those skilled in the art can change or modify the embodiments without departing from the scope and spirit of the present invention.

## Claims

1. A cancer therapeutic instrument comprising:
a catheter type of insertion member having a tube to be inserted into a subject to be treated, and an optical fiber of which a tip end is adapted to be disposed inside said tube; and
a light source for emitting a predetermined activation light, said light source being arranged to introduce the activation light into said optical fiber,
wherein said tube has a cooling means for cooling a portion in which a tip of said optical fiber inserted into the tube is disposed.

2. An instrument according to claim 1, wherein said cooling means is adapted to supply a cooling medium into said tube and collect the supplied cooling medium.

3. An instrument according to claim 2, wherein said cooling medium is a liquid having a boiling point between between 25 °C and 80 °C.

4. An instrument according to claim 1, wherein said cooling means comprises a hetero conductor junction constituted of at least two types of conductor, and a power source for supplying a current to said hetero conductor junction, and wherein said cooling means cools the portion in which the tip of said optical fiber inserted into the tube is disposed, utilizing Peltier effect involved in the current flowing through the hetero conductor junction.
